(19) Europäisches Patentamt
European Patent Office
Office européen des brevets

(11) Veröffentlichungsnummer: **0 238 003**
**B1**

(12) **EUROPÄISCHE PATENTSCHRIFT**

(45) Veröffentlichungstag der Patentschrift: **20.09.89**

(51) Int. Cl.⁴: **C07C 69/533**, C07C 67/00

(21) Anmeldenummer: **87103752.9**

(22) Anmeldetag: **14.03.87**

(54) **Verfahren zur Herstellung von 4-Pentensäureestern.**

(30) Priorität: **19.03.86 DE 3609139**

(43) Veröffentlichungstag der Anmeldung: **23.09.87 Patentblatt 87/39**

(45) Bekanntmachung des Hinweises auf die Patenterteilung: **20.09.89 Patentblatt 89/38**

(84) Benannte Vertragsstaaten: **BE CH DE FR GB IT LI NL**

(56) Entgegenhaltungen:
**US-A- 3 182 077**

**CHEMICAL ABSTRACTS, Band 82, Nr. 19, 12 Mai 1975, Columbus, Ohio, USA Y.MURAMOTO et al. "Alcoholysis of lactones in the presence of magnesium alkoxide. Preparation of 6-hydroxyhexanoates and 5-hydroxypentanoates" Seiten 505,506, Spalten 2,1, Zusammenfassung Nr. 124 673j) 1985, 39(8), 715-2036(5), 225-31 000**

(73) Patentinhaber: **BASF Aktiengesellschaft, Carl-Bosch-Strasse 38, D-6700 Ludwigshafen(DE)**

(72) Erfinder: **Fischer, Rolf, Dr., Bergstrasse 98, D-6900 Heidelberg(DE)**
Erfinder: **Vagt, Uwe, Dr., Paul-Neumann-Strasse 44, D-6720 Speyer(DE)**

**EP 0 238 003 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Anmeldung betrifft ein Verfahren zur Herstellung von 4-Pentensäureestern durch Umsetzung von 5-Methylbutyrolacton bei Temperaturen von 150 bis 400°C mit Alkoholen in Gegenwart von sauren Katalysatoren.

Es ist bekannt, 3-Pentensäureester bei Temperaturen von 100 bis 150°C in Gegenwart von sauren Ionenaustauschern oder sauren Zeolithen, die Edelmetalle der achten Gruppe des periodischen Systems wie Palladium, Rhodium oder Ruthenium enthalten, zu 4-Pentenestern zu isomerisieren (DE-OS 33 17 163). So erhält man z.B. ausgehend von 3-Pentensäuremethylester (70 % trans, 30 % cis) Isomerengemische, die neben unumgesetztem 3-Pentensäuremethylester und geringen Mengen 2-Pentenestern 8 Gew.-% des gewünschten 4-Pentensäuremethylesters enthalten. Der 4-Pentenester läßt sich aus derartigen Isomerengemischen durch eine Azeotropdestillation mit Wasser abtrennen (DE-OS 34 12 295). Nachteilig an dieser Vorgehensweise ist, daß sich der 4-Pentenestergehalt der Isomerengemische nicht wesentlich über 8 Gew.-% steigern läßt. Die Folge ist, daß für die Abtrennung des 4-Pentenesters und die Rückführung der 2- und 3-Pentenester ein beträchtlicher Destillationsaufwand entsteht.

Es bestand daher die technische Aufgabe, ein Verfahren zu entwickeln, bei dem höhere 4-Pentenester-Gehalte im Pentenester-Isomerengemisch erzielt werden.

Diese Aufgabe wird gelöst in einem Verfahren zur Herstellung von 4-Pentensäureestern der Formel

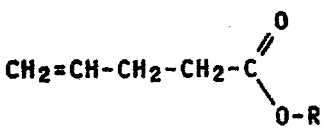

I,

in der R einen Alkylrest mit 1 bis 6 Kohlenstoffatomen bedeutet, bei dem man 5-Methylbutyrolacton der Formel

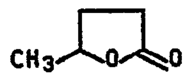

II

mit Alkoholen der Formel

R–OH (III),

in der R die obengenannte Bedeutung hat, bei einer Temperatur von 150 bis 400°C in Gegenwart von sauren Katalysatoren umsetzt.

Die Bildung von Pentenestern und insbesondere 4-Pentenestern bei der Umsetzung von 5-Methylbutyrolacton mit Alkoholen in Gegenwart von sauren Katalysatoren ist überraschend und wurde bisher nicht beschrieben. So wurden bei dem Versuch, Butyrolacton unter den erfindungsgemäßen Bedingungen zu 3-Butencarbonsäuremethylester zu spalten, nur geringe Mengen des gewünschten Esters gefunden (siehe Vergleichsbeispiel 1).

Es war weiterhin nicht vorauszusehen, daß bei der Spaltung von 5-Methylbutyrolacton mit Alkoholen in Gegenwart saurer Katalysatoren neben cis-und trans-3-Pentensäureestern bis zu 38 % 4-Pentensäureester (bezogen auf die Summe aller Pentenester) und lediglich rund 10 % 2-cis-Pentensäureester (bezogen auf 4-Pentensäureester) gebildet werden.

Bemerkenswert ist auch, daß die Lactonspaltung zu Alkencarbonsäureestern gegenüber der ebenfalls sauer katalysierten Etherbildung aus den beteiligten Alkoholen bevorzugt ist.

Die erfindungsgemäße Umsetzung läßt sich für den Fall der Herstellung von 4-Pentensäuremethylester durch die folgende Formelgleichung wiedergeben:

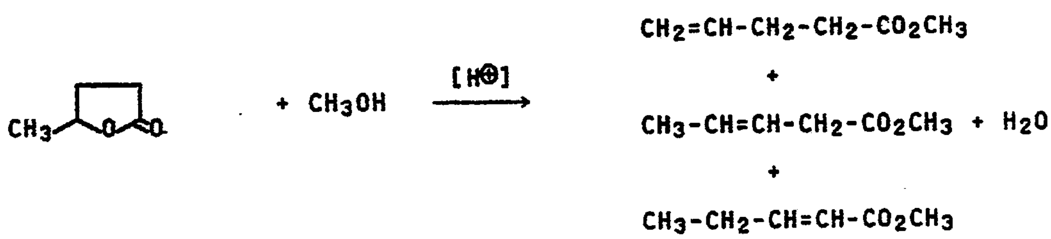


Neben 4-Pentensäuremethylester werden 3- und 2-Pentensäuremethylester gebildet, die mit Ausnahme von 2-cis-Pentensäuremethylester destilliert von 4-Pentensäuremethylester abgetrennt werden können.

Geeignete Alkohole der Formel III sind beispielsweise Methanol, Ethanol, n-Propanol, i-Propanol, tert.-Butanol, n-Butanol, i-Butanol, sec.-Butanol, n-Pentanol, n-Hexanol verwendet. Besonders geeignet sind Methanol, Ethanol und Propanole.

Das Molverhältnis von 5-Methylbutyrolacton II zu Alkohol III beträgt vorteilhaft 1 : 0,5 bis 1 : 10, insbesondere 1 : 1 bis 1 : 5.

Die Umsetzung führt man bei einer Temperatur von 150 bis 400°C, vorzugsweise 200 bis 400°C, insbesondere 250 bis 350°C durch. Vorteilhaft hält man Drücke von 1 bis 100 bar, insbesondere 1 bis 10 bar ein.

Geeignete saure Katalysatoren sind z.B.sauer wirkende Oxide von Elementen der III. und IV. Hauptgruppe sowie der IV. bis VI. Nebengruppe des Periodischen Systems, ferner Protonen- oder Lewis-Säuren.

Die Umsetzung kann diskontinuierlich oder kontinuierlich als Festbettreaktion mit Festbettkatalysatoren, beispielsweise in Sumpf- oder Rieselfahrweise in der Flüssig- oder Gasphase z.B. im Wirbelbett oder aber mit in der Flüssigphase suspendierten Festbettkatalysatoren durchgeführt werden.

Als saure Katalysatoren eignen sich beispielsweise heterogene Katalysatoren wie z.B. Siliciumdioxid in Form von Kieselgel, Kieselgur, Quarz, weiterhin Titandioxid, Zirkondioxid, Phosphorpentoxid, Vanadiumpentoxid, Bortrioxid, Aluminiumoxid, Chromoxide, Molybdänoxide, Wolframoxide oder Gemische derselben. Geeignet sind auch Zeolithe, insbesondere A-, X-, Y-Zeolithe oder Pentasil-Zeolithe in der sauren Form.

Setzt man 5-Methylbutyrolacton an derartigen Festbettkatalysatoren in der Gasphase um, so arbeitet man vorteilhaft mit einer Katalysatorbelastung von 0,1 bis 10, insbesondere von 0,1 bis 5 g 5-Methylbutyrolacton pro g Katalysator und Stunde.

Es ist weiterhin möglich, in der Flüssigphase homogen gelöste saure Katalysatoren zu verwenden.

Geeignet sind z.B. Mineralsäuren, wie Schwefelsäure, Phosphorsäure, Salzsäure, Salpetersäure, Bromwasserstoffsäure, oder Sulfonsäuren, wie Benzolsulfonsäure oder p-Toluolsulfonsäure. Das Molverhältnis von 5-Methylbutyrolacton zu Mineralsäure beträgt beispielsweise 1 : 0,001 bis 1 : 1, insbesondere 1 : 0,01 bis 1 : 0,1.

Die Umsetzung von 5-Methylbutyrolacton II mit Alkoholen in der Flüssigphase wird beispielsweise so durchgeführt, daß man ein Gemisch aus II und dem jeweiligen Alkohol III in Gegenwart eines suspendierten Festbettkatalysators oder eines homogen gelösten Katalysators auf die gewünschte Reaktionstemperatur erhitzt. Nach Ablauf der notwendigen Reaktionszeit wird das Reaktionsgemisch abgekühlt und der saure Katalysator, z.B. durch Filtration oder Neutralisation, entfernt Das Reaktionsgemisch wird anschließend zur Gewinnung der gewünschten 4-Pentensäureester fraktionierend destilliert.

Eine bevorzugte Ausführungsform des erfindungsgemäßen Verfahrens in der Gasphase sieht z.B. so aus, daß man ein Gemisch aus 5-Methylbutyrolacton und dem jeweiligen Alkohol zunächst verdampft und dann, gegebenenfalls zusammen mit einem Inertgas wie Stickstoff, Kohlendioxid oder Argon, bei der gewünschten Reaktionstemperatur gasförmig über einen fest angeordneten insbesondere in auf- und abwirbelnder Bewegung befindlichen Katalysator leitet. Der Reaktionsaustrag wird mittels geeigneter Kühlvorrichtung kondensiert und anschließend durch fraktionierende Destillation aufgearbeitet. Hierbei ist es empfehlenswert, zunächst das Pentensäureestergemisch (Siedepunkt der Methylester z.B. 129 bis 143°C/1013 mbar) vom gegebenenfalls unumgesetzten 5-Methylbutyrolacton (Siedepunkt 207 bis 208°C/1013 mbar) abzudestillieren, um dann den 4-Pentensäureester von den 2- und 3-Pentensäureestern abzutrennen. Zurückgewonnenes 5-Methylbutyrolacton wird zweckmäßig in die Lacton-Spaltung zurückgeführt.

2- und 3-Pentensäureester lassen sich nach Verseifung zu den entsprechenden Pentensäuren in Gegenwart von Schwefelsäure als Katalysator wieder in 5-Methylbutyrolacton verwandeln (R.P. Linstead, J. Chem. Soc. <u>1932</u>, Seiten 115 - 129).

Das erfindungsgemäße Verfahren zur Herstellung von 4-Pentensäureestern besitzt gegenüber der Isomerisierung von 3-Pentenestern zu 4-Pentenestern der Vorteil, daß höhere 4-Pentenestergehalte im Pentenester-Isomerengemisch erreicht werden. Hierdurch gestaltet sich die Abtrennung der 4-Pentenester günstiger. Ein weiterer Vorteil besteht darin, daß anstelle von Edelmetallkatalysatoren, die Palladium, Ruthenium oder Rhodium enthalten, Metalloxide von Nichtedelmetallen als Katalysatoren verwendbar sind.

Die nach dem erfindungsgemäßen Verfahren erhältlichen 4-Pentensäureester stellen wertvolle Zwischenprodukte dar, die sich durch Niederdruck-Hydroformylierung in Gegenwart von Rh-Verbindungen als Katalysatoren in 5-Formylvaleriansäureester umwandeln lassen (DE-OS 33 17 164). 5-Formylvaleriansäureester können durch aminierende Hydrierung und Cyclisierung der entstehenden Aminocapronester ohne Ammonsulfat-Anfall zu Caprolactam umgesetzt werden.

Das Verfahren nach der Erfindung sei an folgenden Beispielen veranschaulicht.

<u>Beispiel 1</u>

Pro Stunde wurde ein Gemisch aus 8,3 g 5-Methylbutyrolacton und 2,7 g Methanol (Molverhältnis 1 : 1) in einen Verdampfer gepumpt und von dort gasförmig zusammen mit 3 Litern Stickstoff bei 300°C über 5 g eines Al@O#-Katalysators geleitet. Die gasförmigen Reaktionsausträge wurden kondensiert, gewogen und gaschromatographisch analysiert. Tabelle 1 zeigt die Zusammensetzung der Reaktionsausträge nach verschiedenen Reaktionszeiten. Der Lacton-Umsatz lag demnach bei rund 25 %, das Verhältnis von 4-Pentensäuremethylester zur Summe aller gebildeten Pentenester bei 33 bis 36 %.

Im Zeitraum zwischen 916 und 1008 Stunden Reaktionszeit wurden 970 g Lacton/-Methanol-Gemisch (732 g Lacton, 7,31 Mole) über den Katalysator geleitet und 906 g Reaktionsaustrag kondensiert. Durch fraktionierende Destillation an einer Drehbandkolonne wurden hieraus 583 g unumgesetztes 5-Methylbutyrolacton (5,82 Mole) und ein Gemisch aus 57 g 4-Pentensäuremethylester (0,5 Mole), 63 g cis + trans-3-Pentensäuremethylester, (0,555 Mole), 6 g 2-cis-Pentensäuremethylester (0,055 Mole) und 39 g 2-trans-Pentensäuremethylester (0,34 Mole) erhalten. Der Umsatz betrug demnach 20 %, die 4-Pentensäuremethylester-Selektivität (2-cis-PSE als Verlust gewertet) 90 %, das Verhältnis von 4-Pentensäuremethylester zur Summe aller gebildeten Pentenester 34,5 %. Als nicht destillierbarer Rückstand der 906 g Reaktionsaustrag wurden lediglich 3 g gefunden.

Vergleichsbeispiel 1

Wurde Beispiel 1 mit Butyrolacton anstelle von 5-Methylbutyrolacton bei 300°C und einem Molverhältnis von Lacton zu Methanol von 1 zu 3 wiederholt, so wurde nach 3 Stunden Reaktionszeit ein Reaktionsaustrag erhalten, der zu 94 % aus unumgesetztem Butyrolacton, zu 3,8 % aus cis-und trans-2-Butensäuremethylester und nur zu 0,6 % aus dem erwünschten 3-Butensäuremethylester bestand.

Beispiele 2 bis 6

Unter gleichen Bedingungen wie in Beispiel 1 angegeben, wurden 10 g eines 5-Methylbutyrolacton/Methanol-Gemisches des in Tabelle 2 angegebenen Molverhältnisses gasförmig über 5 g des jeweiligen Katalysators geleitet. Die Zusammensetzung der Reaktionsausträge ist in der Tabelle angegeben.

Tabelle 1

4–Pentensäuremethylester (4–PSE) durch Spaltung von 5–Methylbutyrolacton mit Methanol

| Reaktionszeit [h] | Flächen-% (ohne $CH_3OH$)[1)] 4–PSE | Σ3–PSE | 2–cis–PSE | 2–tr.–PSE | 5–Methylbutyrolacton | Sonstige | $\frac{4\text{–PSE}}{\Sigma\,\text{PSE}} \times 100$ |
|---|---|---|---|---|---|---|---|
| 25 | 10,7 | 12,3 | 1,2 | 7,1 | 65,0 | 3,7 | 34,2 |
| 118 | 8,5 | 9,0 | 0,8 | 5,1 | 74,7 | 1,9 | 36,3 |
| 212 | 8,4 | 9,0 | 0,8 | 5,1 | 75,6 | 1,1 | 36,1 |
| 332 | 9,0 | 9,5 | 0,9 | 5,4 | 73,9 | 1,3 | 36,3 |
| 550 | 8,4 | 10,0 | 0,9 | 5,9 | 72,9 | 1,9 | 33,3 |
| 646 | 8,6 | 9,5 | 0,8 | 5,5 | 73,3 | 2,3 | 35,2 |
| 742 | 8,2 | 9,3 | 0,8 | 5,4 | 74,7 | 1,6 | 34,6 |
| 838 | 9,0 | 10,6 | 0,9 | 6,1 | 70,5 | 2,9 | 33,8 |
| 934 | 9,5 | 10,0 | 0,8 | 5,8 | 73,0 | 1,9 | 33,9 |
| 1006 | 8,0 | 9,9 | 0,8 | 5,6 | 73,7 | 2,0 | 32,9 |

1) GC-Kapillarsäule Silar 5 CP, 25 m
PSE = Pentensäuremethylester

Tabelle 2

| Beisp. | Katalysator | Reaktionstemp. [°C] | Molverh. Lacton: $CH_3OH$ | Reaktionszeit [h] | Fl.-% (ohne $CH_3OH$) 4-PSE | Σ3-PSE | 2-cis-PSE | 2-tr.-PSE | 5-Methylbutyrolacton | Sonstige | $\frac{4\text{-PSE}}{\Sigma\,\text{PSE}} \times 100$ |
|---|---|---|---|---|---|---|---|---|---|---|---|
| 2 | $Al_2O_3$/$SiO_2$ *) | 300 | 1:1 | 2 | 8,0 | 30,9 | 2,9 | 14,4 | 35,5 | 8,3 | 14 |
| 3 | $TiO_2$ | 400 | 1:1 | 2 | 17,0 | 16,7 | 1,7 | 9,2 | 51,5 | 3,9 | 38 |
| 4 | $B_2O_3$/$Al_2O_3$ **) | 300 | 1:1 | 6 | 4,4 | 8,8 | 0,6 | 4,1 | 79,9 | 2,2 | 25 |
| 5 | $SiO_2$ | 300 | 1:1 | 6 | 9,1 | 14,0 | 0,7 | 3,8 | 71,0 | 1,4 | 33 |
| 6 | $Al_2O_3$ (D 10-10) | 300 | 1:4 | 7 | 14,9 | 15,4 | 1,8 | 10,3 | 53,2 | 4,4 | 35 |

*) 25% $Al_2O_3$, 75% $SiO_2$ **) 45% $B_2O_3$, 55% γ-$Al_2O_3$

Tabelle 2: 4-Pentensäuremethylester (4-PSE) durch Spaltung von 5-Methylbutyrolacton mit Methanol in Gegenwart verschiedener Katalysatoren

## Patentansprüche

1. Verfahren zur Herstellung von 4-Pentensäureestern der Formel

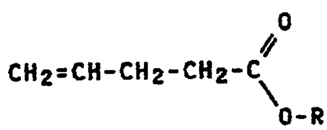

I,

in der R einen Alkylrest mit 1 bis 6 Kohlenstoffatomen bedeutet, dadurch gekennzeichnet, daß man 5-Methylbutyrolacton der Formel

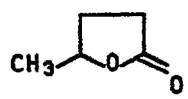

II,

mit Alkoholen der Formel

R-O-H (III),

in der R die obengenannte Bedeutung besitzt, bei Temperaturen von 150 bis 400°C, in Gegenwart von sauren Katalysatoren umsetzt.

2. Verfahren nach Anspruch 1, dadurch gekennzeichnet, daß man als saure Katalysatoren Siliciumdioxid, Aluminiumoxid, Titandioxid, Bortrioxid oder Gemische derselben verwendet.

3. Verfahren nach den Ansprüchen 1 und 2, dadurch gekennzeichnet, daß man 5-Methylbutyrolacton und Alkohole der Formel III im Molverhältnis von 1 : 0,5 bis 1 : 10 einsetzt.

4. Verfahren nach den Ansprüchen 1 bis 3, dadurch gekennzeichnet, daß man eine Temperatur von 200 bis 400°C einhält.

5. Verfahren nach den Ansprüchen 1 bis 4, dadurch gekennzeichnet, daß man die Umsetzung in der Gasphase durchführt.

6. Verfahren nach Anspruch 5, dadurch gekennzeichnet, daß man eine Katalysatorbelastung von 0,1 bis 10 g 5-Methylbutyrolacton pro Gramm Katalysator und Stunde einhält.

## Claims

1. A process for the preparation of a 4-pentenoate of the formula

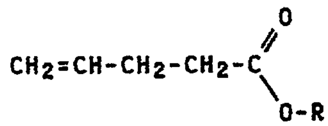

I.

where R is alkyl of 1 to 6 carbon atoms, wherein 5-methyl-butyrolactone of the formula

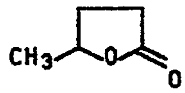

II.

is reacted with an alcohol of the formula

R–O–H (III),

where R has the above meaning, at from 150 to 400°C in the presence of an acidic catalyst.

2. A process as claimed in claim 1, wherein silica, alumina, titanium dioxide, boron trioxide or a mixture of these is used as the acidic catalyst.

3. A process as claimed in either of claims 1 and 2, wherein 5-methyl-butyrolactone and an alcohol of the formula III are used in a molar ratio of from 1 : 0.5 to 1 : 10.

4. A process as claimed in any of claims 1 to 3, wherein a temperature of from 200 to 400°C is maintained.

5. A process as claimed in any of claims 1 to 4, wherein the reaction is carried out in the gas phase.

6. A process as claimed in claim 5, wherein a space velocity of from 0.1 to 10 g of 5-methylbutyrolactone per g of catalyst per hour is maintained.

## Revendications

1. Procédé de préparation d'esters de l'acide 4-penténoïque de formule

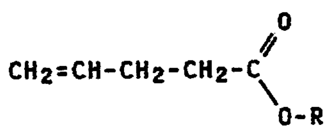

I.

dans laquelle R désigne in radical alkyle contenant 1 à 6 atomes de carbone, caractérisé en ce que l'on fait réagir la 5-méthylbutyrolactone de formule

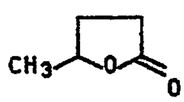

II.

avec des alcools de formule

R–O–H (III)

dans laquelle R a la signification sus-indiquée, à des températures de 150 à 400°C, en présence de catalyseurs acides.

2. Procédé suivant la revendication 1, caractérisé en ce que l'on utilise, comme catalyseurs acides, le dioxyde de silicium, l'oxyde d'aluminium, le dioxyde de titane, le trioxyde de bore ou des mélanges de ces substances.

3. Procédé suivant les revendications 1 et 2, caractérisé en ce que l'on met en oeuvre la 5-méthylbutyrolactone et les alcools de la formule III dans un rapport molaire de 1 : 0,5 à 1 : 10.

4. Procédé suivant les revendications 1 à 3, caractérisé en ce que l'on maintient une température de 200 à 400°C.

5. Procédé suivant les revendications 1 à 4, caractérisé en ce que l'on effectue la réaction en phase gazeuse.

6. Procédé suivant la revendication 5, caractérisé en ce que l'on maintient une charge du catalyseur de 0,1 à 10 g de 5-méthylbutyrolactone par gramme de catalyseur et par heure.